# EUROPÄISCHE PATENTSCHRIFT

(11) **EP 2 114 359 B1**
(45) Veröffentlichungstag und Bekanntmachung des Hinweises auf die Patenterteilung: **01.08.2018**
(21) Anmeldenummer: 08707438.1
(22) Anmeldetag: 31.01.2008
(51) Int. Cl.: A61K 8/37, A61K 8/39, A61K 8/49, A61Q 17/04

(54) **ORGANISCHE MIKROPIGMENTE IN KOSMETISCHEN LICHTSCHUTZEMULSIONEN**
ORGANIC MICROPIGMENTS IN COSMETIC LIGHT PROTECTIVE EMULSIONS
MICROPIGMENTS ORGANIQUES DANS DES ÉMULSIONS COSMÉTIQUES DE PROTECTION CONTRE LA LUMIÈRE

(30) Priorität: 01.02.2007 DE 102007005333
(43) Veröffentlichungstag der Anmeldung: 11.11.2009
(73) Patentinhaber: Beiersdorf AG, 20253 Hamburg (DE)
(72) Erfinder: Reinecke, Myriam, 22529 Hamburg (DE); Skubsch, Kerstin, 25421 Pinneberg (DE); Wolber, Rainer, 22397 Hamburg (DE); Mundt, Claudia, CH-8037 Zürich (CH)
(86) Internationale Anmeldenummer: PCT/EP2008/000749
(87) Internationale Veröffentlichungsnummer: WO 2008/092676

(56) Entgegenhaltungen:
- EP-A- 1 023 892
- WO-A-03/053391
- WO-A2-2007/071584
- DE-A1- 19 802 205
- GB-A- 2 412 866
- GB-A- 2 433 439
- US-A1- 2006 018 846

## Beschreibung

Die vorliegende Erfindung betrifft eine kosmetische O/W-Emulsion enthaltend ein O/W-Emulgatorsystem und partikuläre organische Lichtschutzfilterpigmente.
Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.
Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der deutschen Kosmetikverordnung zusammengefasst.
Eine besondere Form von UV-Lichtschutzfiltersubstanzen stellen die Mikropigmente dar. Die UV-Schutzwirkung der Mikropigmente beruht auf den physikalischen Effekten der Reflexion und Lichtstreuung. In kosmetischen Zubereitungen werden als Mikropigmente fast ausschließlich anorganische Mikropigmente aus Titandioxid, Zinkoxid oder Mischoxiden mit zum Beispiel Eisenoxiden eingesetzt.
Die Vorteile von Mikropigmenten als UV-Filtersubstanz in kosmetischen Zubereitungen liegen vor allem darin begründet, dass die Pigmente im Gegensatz zu gelöst oder flüssig vorliegenden, nicht in die Haut penetrieren. Das Auftreten von allergischen Reaktionen ist damit ausgeschlossen.

Nachteilig am Stande der Technik ist jedoch der Umstand, dass Mikropigmente nur schwer stabil in kosmetische Zubereitungen einzuarbeiten sind. Da die Mikropigmente mit Dispergierhilfsmitteln versehen sind, verändert sich die Emulsion. Die Lagerstabilität ist also gering. Besonders instabil sind dabei Öl-in-Wasser-Emulsionen (O/W-Emulsionen) auf der Basis des Emulgators Glycerylstearatcitrat. In derartige Zubereitungen lassen sich nach dem Stande der Technik keine organischen Lichtschutzfilterpigmente lager-, temperatur- und transportstabil einarbeiten. O/W-Emulsionen auf der Basis von Glycerylstearatcitrat weisen jedoch besonders vorteilhafte kosmetische Eigenschaften wie beispielsweise ein angenehmes Hautgefühl auf.

Es war daher die Aufgabe der vorliegenden Erfindung, die Probleme des Standes der Technik zu beseitigen und O/W-Emulsionen auf der Basis des Emulgators Glycerylsteratcitrat zu entwickeln, in welche sich organische Lichtschutzfilterpigmente stabil einarbeiten lassen. Die Formulierungen sollten ferner bei Ihrer Anwendung auf der Haut ein über einen längeren Anwendungszeitraum stabiles Absorptionsspektrum mit einer ausgewogenen UV-A/UV-B-Absorptionsbalance aufweisen.

Überraschend gelöst werden die Aufgaben durch eine kosmetische O/W-Emulsion enthaltend
a) Glycerylstearatcitrat,
b) einen oder mehrere Emulgatoren mit einem HLB-Wert von kleiner 8,
c) 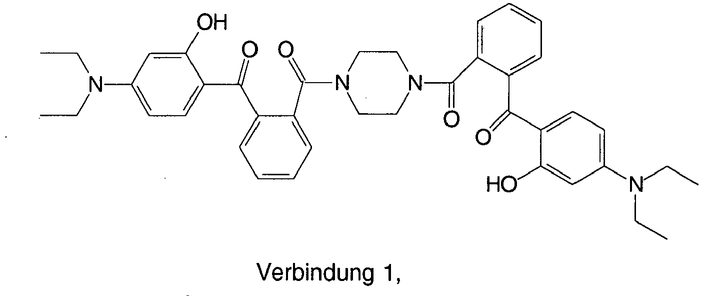 wobei als Emulgator mit einem HLB-Wert von kleiner 8 Sorbitanisosterarat, Cetyldimethiconcopolyol und/oder Polyglyceryl-2 Dipolyhydroxystearat eingesetzt wird.

Überraschend gelöst werden die Aufgaben ferner durch die Verwendung von einem oder mehreren Emulgatoren mit einem HLB-Wert von kleiner 8 zur Stabilisierung partikulärer organischer Lichtschutzfilter vom Typ Verbindung 1. Diese Verwendung war insbesondere überraschend für O/W-Emulsionen.

Zwar kennt der Stand der Technik die US 2006/018846, WO 03/053391, EP 1023892 und die DE 19802205, doch konnten diese Schriften nicht den Weg zur vorliegenden Erfindung weisen.

Erfindungsgemäß vorteilhafte Ausführungsformen der vorliegenden Erfindung sind dadurch gekennzeichnet, dass die Zubereitung das O/W-Emulgatorsystem in einer Konzentration von 0,5 bis 7 Gewichts-%, bezogen auf das Gesamtgewicht der Zubereitung, enthält.

Glycerylstearatcitrat, (2-Hydroxy-1,2,3-propantricarbonsäure-1,2,3-propantriolmonoocta-decanoat, INCI Glyceryl Stearate Citrate, CAS 39175-72-9) ist der Citronensäureester des Glycerylstearat und u.a. unter der Bezeichnung Imwitor 370 bei der Firma Sasol im Handel erhältlich.

Erfindungsgemäß vorteilhafte Ausführungsformen der vorliegenden Erfindung sind dadurch gekennzeichnet, dass die Zubereitung Glycerylstearatcitrat in einer Konzentration von 0,5 bis 5 Gewichts-% und bevorzugt in einer Konzentration von 0,5 bis 3,0 Gewichts-%, jeweils bezogen auf das Gesamtgewicht der Zubereitung, enthält.

Es ist erfindungsgemäß von Vorteil, wenn die erfindungsgemäße Zubereitung einen oder mehrere Emulgatoren mit einem HLB-Wert von kleiner 8 in einer Gesamtkonzentration von 0,1 bis 3 Gewichts-% und erfindungsgemäß bevorzugt in einer Konzentration von 0,3 bis 1,5 Gewichts-%, jeweils bezogen auf das Gesamtgewicht der Zubereitung, enthält.

Erfindungsgemäß bevorzugt werden Emulgatoren mit einem HLB-Wert von kleiner/gleich 6 , wie z.B.
Sorbitanisosterarat (z.B. Arlacel 60, Uniquema), Cetyldimethiconcopolyol (z.B. Abil EM 90, Goldschmidt), Polyglyceryl-2 Dipolyhydroxystearat (Dehymuls PGPH, Cognis) eingesetzt.

Es ist erfindungsgemäß bevorzugt, wenn das Gewichtsverhältnis von O/W Emulgator zur Gesamtmenge an Emulgatoren mit einem HLB-Wert von kleiner 8 von 1 : 1 bis 20 : 1 und besonders bevorzugt, wenn das Gewichtsverhältnis von Glycerylstearatcitrat zur Gesamtmenge an Emulgatoren mit einem HLB-Wert von kleiner 8 von 1,5 :1 bis 8 : 1 beträgt.

Es ist erfindungsgemäß von Vorteil, wenn die erfindungsgemäße Zubereitung organische Lichtschutzfilterpigmente der Verbindung 1 in einer Gesamtkonzentration von 0,1 bis 30 Gewichts-% und erfindungsgemäß bevorzugt in einer Konzentration von 0,5 bis 15 Gewichts-%, jeweils bezogen auf das Gesamtgewicht der Zubereitung, enthält.

Es ist erfindungsgemäß vorteilhaft, Verbindung 1 in Form einer wässrigen Dispersion zuzusetzen. Derartige Dispersionen enthalten Verbindung 1 in der Regel in einem Gehalt von 45 bis 50 Gewichts-%, bezogen auf das Gesamtgewicht der Dispersion. Als Dispergierhilfen enthalten solche Dispersionen in der Regel die üblichen Dispergiermittel, beispielsweise Plantacare 2000 UP (Cognis), Amphisol K (DSM), Texapon K14S Spezial (Cognis)Rhodicare S (C.H. Erbslöh KG), Silfoam SE2 (Wacker), Butylenglycol, Zitronensäure, NaOH.

Vorteilhafte Ausführungsformen der vorliegenden Erfindung sind dadurch gekennzeichnet, dass die erfindungsgemäße Zubereitung einen oder mehrere Dispergiermittel enthält. Als Dispergierhilfe/Dispergiermittel sind z. B C8-C16 Alkylpolyglucosid und amphiphile Polymere wie sind in EP 1093796 B1 beschrieben sind, erfindungsgemäß vorteilhaft einsetzbar.

Erfindungsgemäß bevorzugt werden dabei ein oder mehrere Dispergiermittel gewählt aus der Gruppe der Alkylpolyglycoside, Xanthangummi.

Es ist erfindungsgemäß vorteilhaft, wenn die Zubereitung oder Verwendung gemäß der vorliegenden Erfindung, weitere UV-Filtersubstanzen enthält.

Ferner kann die erfindungsgemäße Zubereitung vorteilhaft UV-Lichtschutzfiltersubstanzen auf der Basis anorganischer Pigmente enthalten. Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Bariumsulfat (BaSO₄) oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegender Erfindung auch in Form kommerziell erhältlicher öliger oder wäßriger Vordispersionen zur Anwendung kommen. Diesen Vordispersionen können vorteilhaft Dispergierhilfmittel und/oder Solubilisationsvermittler zugesetzt sein.

Die erfindungsgemäßen Titandioxide zeichnen sich durch eine Primärpartikelgröße zwischen 10 nm bis 200 nm aus, wobei Partikelgrößen von 10 nm bis 100 nm erfindungsgemäß bevorzugt sind.

| **Handelsname** | **Coating** | **zusätzliche Bestandteile der Vordispersion** | **Hersteller** |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KgaA |
| Eusolex TS | Alumina, Stearinsäure | - | Merck KgaA |
| Eusolex T-AVO | Silica | | Merck KgaA |
| Titandioxid P25 | None | - | Degussa |
| Titandioxid T805 (Uvinul TiO₂) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |
| Titandioxid T-817 (Eisen/TitanMisch oxid) | Eisenoxid | | Degussa |

Im Sinne der vorliegenden Erfindung sind besonders bevorzugte Titandioxide das MT-100 Z und MT-100 TV von Tayca Corporation, Eusolex T-2000 und Eusolex T-AVO von Merck und das Titandioxid T 805 von Degussa und das Eisen/Titandmischoxid Titandioxid T817 von Degussa.

Zinkoxide können im Sinne der vorliegenden Erfindung auch in Form kommerziell erhältlicher öliger oder wäßriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Vorteilhafte weitere UV-Filtersubstanzen im Sinne der vorliegenden Erfindung sind beispielsweise die im folgenden genannten, welche in der Wasser- und/oder der Ölphase vorliegen können.

Vorteilhafte bei Raumtemperatur flüssige UV-Filtersubstanzen im Sinne der vorliegenden Erfindung sind Homomenthylsalicylat (INCI: Homosalate), 2-Ethylhexyl-2-hydroxybenzoat (2-Ethylhexylsalicylat, Octylsalicylat, INCI: Octyl Salicylate), 2-Ethylhexyl-2-cyano-3,3-diphenyl-acrylat (INCI: Octocrylene) und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan / Dimethylsiloxan - Copolymer welches beispielsweise unter der Handelsbezeichnung Parsol® SLX bei Hoffmann La Roche erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen im Sinne der vorliegenden Erfindung sind Hydroxybenzophenone, welche sich durch die folgende Strukturformel auszeichnen: 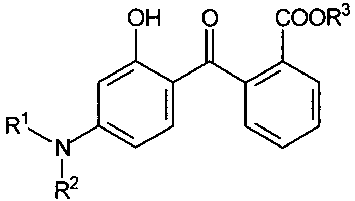 worin
R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
R³ einen C₁-C₂₀-Alkyl Rest bedeutet.

Ein besonders vorteilhaftes Hydroxybenzophenon im Sinne der vorliegenden Erfindung ist das 2-(4'-Diethylamino-2'-hydoxybenzoyl)-benzoesäurehexylester (auch: Aminobenzophenon), welches sich durch folgende Struktur auszeichnet: 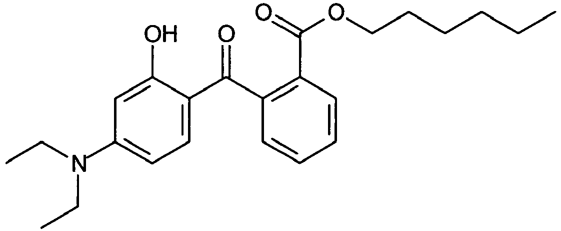 und unter dem Uvinul A Plus bei der Fa. BASF erhältlich ist.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornyliden-methyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)-ester, 4-(Dimethylamino)benzoesäureamylester;
- Ester der Benzalmalonsäure, vorzugsweise 4-Methoxybenzalmalonsäuredi(2-ethylhexyl)-ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter,
- Homomenthylsalicylat (INCI: Homosalate) und
- 2-Ethylhexyl-2-hydroxybenzoat (2-Ethylhexylsalicylat, Octylsalicylat, INCI: Octyl Salicylate).

Besonders vorteilhafte UV-Filtersubstanzen sind:
- Phenylen-1,4-bis-(2-benzimidazyl)-3,3'-5,5'-tetrasulfonsäure-bis-natriumsalz (INCI: Bisimidazylate, Handelsname: Neoheliopan AP),
- 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0, erhältlich bei 3V Sigma unter der Handelsbezeichnung Uvasorb® K2A) in gelöster Form,
- Dioctylbutylamidotriazon (INCI: Diethylhexyl Butamido Triazone, Handelsname: Uvasorb HEB),
- 2-(4'-Diethylamino-2'-hydoxybenzoyl)-benzoesäurehexylester (auch: Aminobenzophenon) (INCI: Diethylamino Hydroxybenzoyl Hexyl Benzoat, Handelsname: Uvinul A plus),
- (3Z)-1,7,7-trimethyl-3-(4-methylbenzylidene)bicyclo[2.2.1]heptan-2-one (INCI: 4-Methylbenzylidene Campher, Handelsname: Eusolex 6300),
- 2-Ethylhexyl-2-cyano-3,3-diphenylacrylat (INCI: Octocrylene, Handelsname: Uvinul N-539),
- Benzol-1,4-di(2-oxo-3-bornylidenmethyl-10-sulfonsäure) (INCI: Terephtalidene Dicampher Sulfonsäure, Handelsname: Mexoryl SX),
- 2-(2H-benzotriazol-2-yl)-4-methyl-6-[2-methyl-3-[1,3,3,3-tetramethyl-1-[(trimethylsilyl)oxy]di-siloxanyl]propyl]-phenol (INCI: Drometrizole Trisiloxane, Handelsname: Mexoryl XL).
- Titandioxide

Ganz besonders vorteilhafte UV-Filtersubstanzen im Sinne der vorliegenden Erfindung sind:
- 2-Ethylhexyl-4-methoxycinnamat (INCI: Octyl Methoxycinnamat, Handelsname: Parsol MCX),
- 4-(tert.-Butyl)-4'-methoxydibenzoylmethan (INCI: Butylmethoxydibenzoylmethan, Handelsname: Parsol 1789),
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (Handelsname: Eusolex 232).
- 2,4-Bis-{[4-(2-ethyl-hexyloxy)-2-hydroxy]-phenyl}-6-(4-methoxyphenyl)-1,3,5-triazin (INCI: Aniso Triazin, erhältlich unter dem Handelsnamen Tinosorb S) in gelöster Form,
- 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird) in gelöster Form,
- Dioctylbutylamidotriazon (INCI: Diethylhexyl Butamido Triazone, Handelsname: Uvasorb HEB).

Diese zusätzlichen UV-Filtersubstanzen können erfindungsgemäß vorteilhaft in einer Gesamtkonzentration von 0,01 bis 20 Gewichts-% und erfindungsgemäß bevorzugt in einer Gesamtkonzentration von 0,5 bis 15 Gewichts-%, jeweils bezogen auf das Gesamtgewicht er Zubereitung in dieser enthalten sein.

Die Ölphase der erfindungsgemäßen Zubereitungen wird vorteilhaft gewählt aus der Gruppe der polaren Lipide mit einer Polarität ≤ 35 mN/m Besonders vorteilhafte Lipide im Sinne der vorliegenden Erfindung sind alle nativen Lipide, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl und dergleichen sowie die im folgenden aufgelisteten.

| **Hersteller** | **Handelsname** | **INCI-Name** | **Polarität mN/m** |
|---|---|---|---|
| Cognis | Cetiol OE | Dicaprylylcarbonat | 31,7 |
| Cognis | Cetiol CC | Dicaprylylether | 30,9 |
| Stearinerie Dubois Fils | DUB VCI 10 | Isodecyl Neopentanoate | 29,9 |
| Lipo Chemicals Inc | Liponate TDTM | Tridecyl Trimellitate | 27,2 |
| Wacker | Wacker AK 100 | Dimethicone | 26,9 |
| Henkel Cognis | Eutanol G | Octyldodecanol | 24,8 |
| | Macadamia Nut Oil | | 22,1 |
| Bayer AG, Dow Corning | Silikonöl VP 1120 | Phenyl Trimethicone | 22,7 |
| Henkel Cognis | Isopropylstearat | Isopropyl Stearate | 21,9 |
| WITCO, Goldschmidt | Finsolv TN | C12-15 Alkyl Benzoate | 21,8 |
| Dr. Straetmans | Dermofeel BGC | Butylene Glycol Dicaprylate/Dicaprate | 21,5 |
| Unichema Huels | Miglyol812 | Caprylic/Capric Triglyceride | 21,3 |
| Henkel Cognis | Cetiol B | Dibutyl Adipate | 14,3 |
| Condea Augusta S.P.A. | Cosmacol ELI | C12-13 Alkyl Lactate | 8,8 |
| Condea Augusta S.P.A. | Cosmacol ETI | Di-C12/13 Alkyl Tartrate | 7,1 |
| Henkel Cognis | Myritol 331 | Cocoglycerides | 5,1 |
| Symrise | Corapan TQ | Diethylhexylnaphthalat | n.b. |
| ISP | X-Tend 226 | 2-Phenylethylbenzoat | n.b. |
| Ajinomoto | Eldew SL 205 | Isopropyl Lauroyl Sarcosinate | n.b. |

Von den Kohlenwasserstoffen sind insbesondere Paraffinöl sowie weitere hydrierte Polyolefine wie hydriertes Polyisobutene, Squalan und Squalen vorteilhaft im Sinne der vorliegenden Erfindung zu verwenden.

Die Gehalt der Lipide wird vorteilhaft kleiner als 50 Gew.-% gewählt, bevorzugt zwischen 1 und 40 Gew.-%, insbesondere bevorzugt zwischen 5 und 15 Gew.-%, jeweils bezogen auf das Gesamtgewicht der Zubereitung.

Falls die Lipidphase öllösliche UV-Filtersubstanzen enthält, ist es vorteilhaft den Gehalt der Lipidphase kleiner als 80 Gew.-% zu wählen, bevorzugt zwischen 1 und 40 Gew.-%, insbesondere bevorzugt zwischen 5 und 30 Gew.-%, jeweils bezogen auf das Gesamtgewicht der Zubereitung.

Es kann gegebenenfalls vorteilhaft sein, wenngleich es nicht zwingend ist, wenn die Ölphase der Zubereitungen im Sinne der vorliegenden Erfindung auch unpolare Lipide enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Propandiol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß besonders vorteilhafte Verdickungsmittel sind Permulen TR 1, TR 2, Carbopol 1328, Aristoflex AVC.

Die erfindungsgemäßen kosmetischen Zubereitungen können wie üblich zusammengesetzt sein. Besonders vorteilhaft im Sinne der vorliegenden Erfindung sind Zubereitungen zur Pflege der Haut: sie können dem kosmetischen Lichtschutz, ferner zur Reinigung oder Pflege der Haut und/oder der Haare und als Schminkprodukt in der dekorativen Kosmetik dienen. Eine weitere vorteilhafte Ausführungsform der vorliegenden Erfindung besteht in After-Sun-Produkten.

Entsprechend ihrem Aufbau können kosmetische Zusammensetzungen im Sinne der vorliegenden Erfindung, beispielsweise verwendet werden als Hautschutzcreme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Erfindungsgemäß vorteilhaft können die erfindungsgemäßen Zubereitungen in Form einer Lotion, insbesondere in Form einer sprühbaren Lotion vorliegen. Neben der Anwendungsform eines Sprays kann die erfindungsgemäße Zubereitung auch zur Tränkung eines Tuches verwendet werden.

Es ist auch vorteilhaft im Sinne der vorliegenden Erfindung, kosmetische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische Zubereitungen, die in der Form eines Sonnenschutzmittels vorliegen.

Zur Anwendung werden die erfindungsgemäßen kosmetischen Zubereitungen in der für Kosmetika üblichen Weise auf die Haut und/oder die Haare in ausreichender Menge aufgebracht.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Ethylhexylglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, Niacinamid, Panthenol, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid) und/oder Licochalcon A.

Licochalcon kann vorteilhaft auch als Bestandteil von pflanzlichen Extrakten, insbesondere von wäßrigen *Radix Glycyrrhizae inflatae,* eingesetzt werden.

Es ist erfindungsgemäß vorteilhaft, wenn die kosmetischen Zubereitungen 0,001 bis 10 Gew.-%, insbesondere 0,05 bis 5 Gew.-%, ganz besonders 0,01 bis 2 Gew.-% an einem Extrakt aus *Radix Glycyrrhizae inflatae* enthalten, jeweils bezogen auf das Gesamtgewicht der Zubereitung.

Ganz besonders vorteilhaft ist es, von einem Extrakt auszugehen, der unter der Bezeichnung Polyol Soluble Licorice Extract PU (INCI-Bezeichnung Glycyrrhiza Inflata) von der Firma Maruzen zu erhalten ist. Der Extrakt aus *Radix Glycyrrhizae inflatae* enthält einen Anteil von ca. 25 % Licochalcone A.

Erfindungsgemäß vorteilhaft kann die erfindungsgemäße Zubereitung einen oder mehrere die Haut aufhellende Stoffe (Depigmentiermittel, "whitening" Substanzen) enthalten, beispielsweise Hydrochinon (1,4-Dihydroxybenzol), Glucocorticoide, Gurkenextrakt, Bärentraubenblätterextrakt, Brunnenkresse-Extrakt, α,w-Dicarbonsäuren (engl. dioic acid) oder Kojic-Säure.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zum Schutz vor ästhetisch unattraktiven Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Zubereitungen gemäß der vorliegenden Erfindung können ferner vorteilhaft auch Selbstbräunungssubstanzen enthalten, wie beispielsweise Dihydroxyaceton und/oder Melaninderivate in Konzentrationen von 1 Gew.-% bis zu 8 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Methylpropandiol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz kosmetischer Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9). Ferner ist es erfindungsgemäß vorteilhaft Bornitrid, Mica , Nylon 12, Covabead LH 85 und/oder Mearlmica SVA einzusetzen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

| **Emulsion** | **A** | **B** |
|---|---|---|
| Glycerylstearatcitrat | 2 | 2 |
| Sorbitanstearat | | 0,5 |
| PEG-7 Hydrogenated Castor Öl | | |
| Polyglyceryl-2 Dipolyhydroxystearat | 0,8 | |
| PEG-30 Dipolyhydroxystearat | | |
| Stearylalkohol | | 2 |
| Cetylalkohol | 1 | |
| Acrylates/C₁₀₋₃₀ Alkyl Acrylat Crosspolymer | 0,2 | 0,1 |
| Carbomer | | |
| Xanthan Gum | | 0,4 |
| C₁₂₋₁₅ Alkyl Benzoat | | |
| C₁₂₋₁₃ Alkyl Tartrat | 3 | |
| Butylenglycol Dicaprylat/Dicaprat | | 5 |
| Dicaprylyl Ether | | |
| Cyclomethicon | | 2 |
| Isohexadecan | | |
| Butylenglycol | 4 | |
| Propylenglykol | | |
| Glycerin | 3 | 8 |
| UVASorb® K2A | | |
| Uvinul A Plus® | 3 | |
| Homosalat | | |
| Phenylbenzimidazol Sulfonsäure | | 2 |
| Octylsalicylat | | |
| Octocrylen | 4 | |
| Bis-Ethylhexyloxyphenol Methoxyphenyltriazin | | 2 |
| 2,2'-Methylen-bis-(6-(2H-benzotriazol-2-yl)-4-(1,1,3,3-tetramethylbutyl)-phenol) | 2 | 1 |
| Verbindung 1 | 3 | 2 |
| C8-C16 Alkylpolyglycosid | 1 | 1 |
| Vitamin E Acetat | | 0,5 |
| Na₂H₂EDTA | | 0,5 |
| Parfüm, Konservierungsmittel | q.s. | q.s. |
| Farbstoffe, usw. | q.s. | q.s. |
| Natriumhydroxid | q.s. | q.s. |
| Wasser | ad 100,0 | ad 100,0 |

| **Emulsion** | **C** | **D** | **E** |
|---|---|---|---|
| Glycerylstearatcitrat | 2 | 2,5 | 2 |
| Sorbitanstearat | | 1 | 0,5 |
| Polyglyceryl-3 Diisostearat | | | |
| Cetyl Dimethicone Copolyol | 0,5 | | |
| Stearylalkohol | 2 | | |
| Cetylalkohol | | 2,5 | 2 |
| Acrylates/C₁₀₋₃₀ Alkyl Acrylat Crosspolymer | | 0,4 | |
| Aristoflex AVC ® | | | 0,2 |
| Xanthan Gum | 0,3 | | 0,2 |
| C₁₂₋₁₅ Alkyl Benzoat | | | |
| Octyldodecanol | | 4 | |
| Butylenglycol Dicaprylat/Dicaprat | 7 | | 5 |
| Diethyl hexylnaphthalat | 2 | | |
| 2-Phenylethyl Benzoat | | 4 | 2 |
| Isodecyl Neopentanoat | | 5 | |
| Isopropylpalmitat | 5 | | |
| Cyclomethicon | | | 6 |
| Propylenglykol | | | |
| Glycerin | 4 | 6 | 10 |
| Ethylhexyltriazon | | 3 | 2 |
| Diethylhexylbutamidotriazon | | 2 | 3 |
| Butyl Methoxydibenzoylmethan | 2 | 3 | |
| Benzophenon-3 | 3 | | 2 |
| Octylsalicylat | 5 | | |
| Eusolex T-AVO ® (Merck) | 2 | 5 | |
| Titandioxid T 805 ® | | | 5 |
| Bis-Ethylhexyloxyphenol Methoxyphenyltriazin | | 2 | |
| 2,2'-Methylen-bis-(6-(2H-benzotriazol-2-yl)-4-(1,1,3,3-tetramethylbutyl)-phenol) | | 4 | |
| 2,4,6-Tris-(biphenyl)-1,3,5-triazin | 6 | 4 | 4 |
| C8-C16 Alkylpolyglycosid | 1 | 1 | 1 |
| 2,4,6-Tris-(terphenyl)-1,3,5-triazin | | | 2 |
| Verbindung 1 | 3 | 2 | 4 |
| Panthenol | | 0,5 | |
| Taurin | 0,1 | | 0,2 |
| Tapiocastärke | | 5 | |
| 8-Hexadecen-1,16-dicarbonsäure | | 0,5 | |
| Parfüm, Konservierungsmittel | q.s. | q.s. | q.s. |
| Farbstoffe, usw. | q.s. | q.s. | q.s. |
| Natriumhydroxid | q.s. | q.s. | q.s. |
| Wasser | ad 100,0 | ad 100,0 | ad 100,0 |

| **Emulsion** | **F** | **G** | **H** | **I** |
|---|---|---|---|---|
| Glycerylstearatcitrat | 2,5 | 3 | 2 | 2 |
| Sorbitanstearat | 0,5 | 1 | | 0,3 |
| Cetyl Dimethicone Copolyol | | | 0,75 | 0,5 |
| Stearylalkohol | | 1,5 | 2 | |
| Cetylalkohol | 1 | | | 1,5 |
| Acrylates/C₁₀₋₃₀ Alkyl Acrylat Crosspolymer | | 0,1 | 0,2 | 0,2 |
| Carbomer | | | | 0,2 |
| Xanthan Gum | 0,4 | 0,3 | | |
| C₁₂₋₁₅ Alkyl Benzoat | 7,5 | | | |
| C₁₂₋₁₃ Alkyl Tartrat | | 4 | | |
| Butylenglycol Dicaprylat/Dicaprat | 5 | 4 | 8 | |
| Dicaprylylcarbonat | 2 | | 2 | |
| Dimethicon | 5 | | 3 | |
| C18-38 Säuretriglycerid | | 1 | | 2 |
| Methylpropandiol | | 5 | 8 | |
| Propylenglykol | | | | 4 |
| Glycerin | 7,5 | 5 | | 3 |
| Ethylhexylmethoxycinnamat | | 5 | | |
| Phenylbenzimidazol Sulfonsäure | | 3 | 2 | |
| NeoHeliopan® AP | | | 2 | |
| Titandioxid T 805 | 5 | 2 | | 2,5 |
| Ethylhexyltriazon | 1,5 | | 3 | 1 |
| Diethylhexylbutamidotriazon | 4 | 3 | | 2 |
| Butyl Methoxydibenzoylmethan | 4,5 | 2 | | 3 |
| Bis Ethylhexyloxyphenol Methoxyphenyltriazin | 3 | | | 1,5 |
| 2,2'-Methylen-bis-(6-(2H-benzotriazol-2-yl)-4-(1,1,3,3-tetramethylbutyl)-phenol) | | 4 | 3 | 6 |
| 2,4,6-Tris-(biphenyl)-1,3,5-triazin | 6 | | 3 | |
| 2,4,6-Tris-(terphenyl)-1,3,5-triazin | | 0,5 | | |
| Verbindung 1 | 2 | 0,5 | 1 | 1,5 |
| C8-C16 Alkylpolyglycosid | 1 | 1 | 1 | 1 |
| Vitamin E Acetat | 0,5 | | | |
| Coenzym Q10 | | | 0,2 | |
| Na₂H₂EDTA | 0,2 | | | |
| Parfüm, Konservierungsmittel | q.s. | q.s. | q.s. | q.s. |
| Farbstoffe, usw. | q.s. | q.s. | q.s. | q.s. |
| Natriumhydroxid | q.s. | q.s. | q.s. | q.s. |
| Wasser | ad 100,0 | ad 100,0 | ad 100,0 | ad 100,0 |

## Patentansprüche

1. Kosmetische O/W-Emulsion enthaltend
a) Glycerylstearatcitrat
b) einen oder mehrere Emulgatoren mit einem HLB-Wert von kleiner 8,
c) 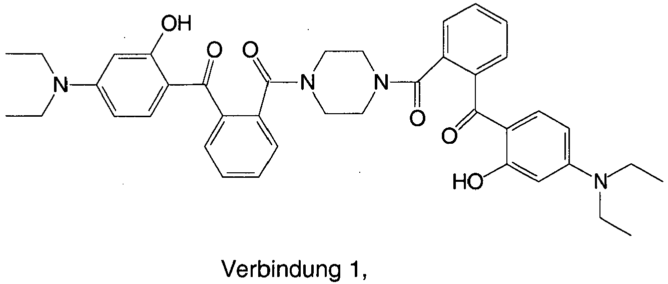 wobei als Emulgator mit einem HLB-Wert von kleiner 8 Sorbitanisosterarat, Cetyldimethiconcopolyol und/oder Polyglyceryl-2 Dipolyhydroxystearat eingesetzt wird.

2. Kosmetische O/W-Emulsion nach Anspruch 1, **dadurch gekennzeichnet, dass** die Zubereitung das O/W-Emulgatorsystem in einer Konzentration von 0,5 bis 7 Gewichts-%, bezogen auf das Gesamtgewicht der Zubereitung, enthält.

3. Kosmetische Emulsion nach einem der vorhergehenden Ansprüche, **dadurch gekennzeichnet, dass** die Zubereitung Glycerylstearatcitrat in einer Konzentration von 0,5 bis 5 Gewichts-%, bezogen auf das Gesamtgewicht der Zubereitung, enthält.

4. Kosmetische Emulsion nach einem der vorhergehenden Ansprüche, **dadurch gekennzeichnet, dass** die Zubereitung einen oder mehrere Emulgatoren mit einem HLB-Wert von kleiner 8 in einer Gesamtkonzentration von 0,1 bis 3 Gewichts-%, bezogen auf das Gesamtgewicht der Zubereitung, enthält.

5. Kosmetische Emulsion nach einem der vorhergehenden Ansprüche, **dadurch gekennzeichnet, dass** die Zubereitung organische Lichtschutzfilterpigmente der Verbindung 1 in einer Gesamtkonzentration von 0,1 bis 30 Gewichts-%, bezogen auf das Gesamtgewicht der Zubereitung, enthält.

6. Kosmetische Emulsion nach einem der vorhergehenden Ansprüche, **dadurch gekennzeichnet, dass** die Zubereitung einen oder mehrere Dispergiermittel enthält.

7. Kosmetische Emulsion nach einem der vorhergehenden Ansprüche, **dadurch gekennzeichnet, dass** das Gewichtsverhältnis von O/W-Emulgatoren zur Gesamtmenge an Emulgatoren mit einem HLB-Wert von kleiner 8 von 1:1 bis 20:1 beträgt.

8. Kosmetische Emulsion nach einem der vorhergehenden Ansprüche, **dadurch gekennzeichnet, dass** ein oder mehrere Dispergiermittel gewählt aus der Gruppe der Alkylpolyglycoside, Xanthangummi.

9. Kosmetische Emulsion nach einem der vorhergehenden Ansprüche, **dadurch gekennzeichnet, dass** sie weitere UV-Lichtschutzfilter enthält.

## Claims

1. Cosmetic O/W emulsion comprising
a) glyceryl stearate citrate
b) one or more emulsifiers with an HLB value of less than 8,
c) 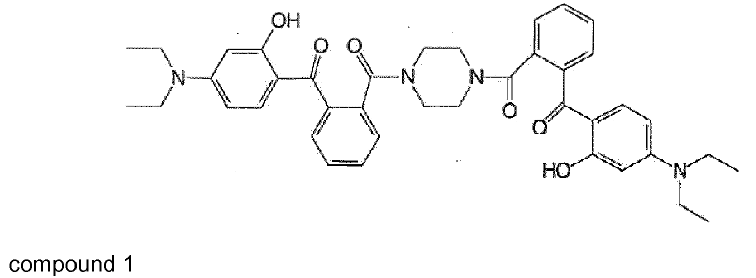 wherein sorbitan isostearate, cetyldimethicone copolyol and/or polyglyceryl-2 dipolyhydroxystearate is/are used as emulsifier with an HLB value of less than 8.

2. Cosmetic O/W emulsion according to Claim 1, **characterized in that** the preparation comprises the O/W emulsifier system in a concentration of from 0.5 to 7% by weight, based on the total weight of the preparation.

3. Cosmetic emulsion according to one of the preceding claims, **characterized in that** the preparation comprises glyceryl stearate citrate in a concentration of from 0.5 to 5% by weight, based on the total weight of the preparation.

4. Cosmetic emulsion according to one of the preceding claims, **characterized in that** the preparation comprises one or more emulsifiers with an HLB value of less than 8 in a total concentration of from 0.1 to 3% by weight, based on the total weight of the preparation.

5. Cosmetic emulsion according to one of the preceding claims, **characterized in that** the preparation comprises organic sunscreen filter pigments of compound 1 in a total concentration of from 0.1 to 30% by weight, based on the total weight of the preparation.

6. Cosmetic emulsion according to one of the preceding claims, **characterized in that** the preparation comprises one or more dispersants.

7. Cosmetic emulsion according to one of the preceding claims, **characterized in that** the weight ratio of O/W emulsifiers to the total amount of emulsifiers with an HLB value of less than 8 is from 1:1 to 20:1.

8. Cosmetic emulsion according to one of the preceding claims, **characterized in that** one or more dispersants selected from the group of alkyl polyglycosides, xanthan gum.

9. Cosmetic emulsion according to one of the preceding claims, **characterized in that** it comprises further UV sunscreen filters.

## Revendications

1. Émulsion H/E cosmétique, contenant
a) du citrate-stéarate de glycéryle
b) un ou plusieurs émulsifiants ayant une valeur HLB inférieure à 8,
c) 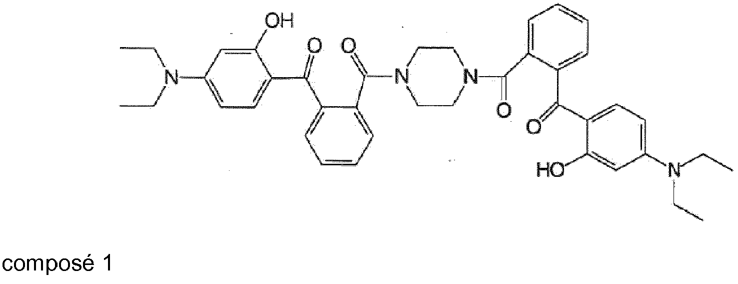 dans laquelle on utilise en tant qu'émulsifiant ayant une valeur HLB inférieure à 8 de l'isostéarate de sorbitanne, du cétyldiméthicone-copolyol et/ou du 2-dipolyhydroxystéarate de polyglycéryle.

2. Émulsion H/E cosmétique selon la revendication 1, **caractérisée en ce que** la préparation contient le système émulsifiant H/E à une concentration de 0,5 à 7 % en poids, par rapport au poids total de la préparation.

3. Émulsion cosmétique selon l'une quelconque des revendications précédentes, **caractérisée en ce que** la préparation contient du citrate-stéarate de glycéryle à une concentration de 0,5 à 5 % en poids, par rapport au poids total de la préparation.

4. Émulsion cosmétique selon l'une quelconque des revendications précédentes, **caractérisée en ce que** la préparation contient un ou plusieurs émulsifiants ayant une valeur HLB (rapport hydrophile-lipophile) inférieure à 8 à une concentration totale de 0,1 à 3 % en poids, par rapport au poids total de la préparation.

5. Émulsion cosmétique selon l'une quelconque des revendications précédentes, **caractérisée en ce que** la préparation contient des pigments filtres photoprotecteurs organiques du composé à une concentration totale de 0,1 à 30 % en poids, par rapport au poids total de la préparation.

6. Émulsion cosmétique selon l'une quelconque des revendications précédentes, **caractérisée en ce que** la préparation contient un ou plusieurs dispersants.

7. Émulsion cosmétique selon l'une quelconque des revendications précédentes, **caractérisée en ce que** le rapport pondéral des émulsifiants H/E à la quantité totale d'émulsifiants ayant une valeur HLB inférieure à 8 vaut de 1:1 à 20:1.

8. Émulsion cosmétique selon l'une quelconque des revendications précédentes, **caractérisée en ce qu'**un ou plusieurs dispersants choisis dans le groupe des alkylpolyglycosides, de la gomme xanthane.

9. Émulsion cosmétique selon l'une quelconque des revendications précédentes, **caractérisée en ce qu'**elle contient d'autres filtres photoprotecteurs UV.
